# EUROPEAN PATENT SPECIFICATION

(11) **EP 1 848 415 B1**
(45) Date of publication and mention of the grant of the patent: **03.04.2013**
(21) Application number: 06720863.7
(22) Date of filing: 17.02.2006
(51) Int. Cl.: A61K 31/135, A61K 38/02, A61P 25/28

(54) **COMBINATION THERAPY WITH GLATIRAMER ACETATE AND RASAGILINE FOR THE TREATMENT OF MULTIPLE SCLEROSIS**
KOMBINATIONSTHERAPIE MIT GLATIRAMERACETAT UND RASAGILIN ZUR BEHANDLUNG VON MULTIPLER SKLEROSE
THERAPIE COMBINEE AVEC DE L'ACETATE DE GLATIRAMERE ET DE RASAGILINE DESTINEE AU TRAITEMENT DE LA SCLEROSE EN PLAQUES

(30) Priority: 17.02.2005 US 654012 P
(43) Date of publication of application: 31.10.2007
(73) Proprietor: TEVA PHARMACEUTICAL INDUSTRIES, LTD., 49131 Petach-Tikva (IL)
(72) Inventor: KREITMAN, Rivka, Maple Glen, Pennsylvania 19002 (US); HAYARDENY, Liat, 69391 Tel-aviv (IL); LEVY, Ruth, 69620 Tel-aviv (IL); BLAUGRUND, Eran, 76449 Rehovot (IL)
(74) Representative: Nachshen, Neil Jacob
(86) International application number: PCT/US2006/005741
(87) International publication number: WO 2006/089164

(56) References cited:
- US-B1- 6 214 791
- US-B1- 6 316 504
- SELA M ET AL: "Glatiramer acetate in the treatment of multiple sclerosis" EXPERT OPINION ON PHARMACOTHERAPY, ASHLEY, LONDON, UK LNKD- DOI:10.1517/14656566.2.7.1149, vol. 2, no. 7, 1 January 2001 (2001-01-01) , pages 1149-1165, XP008096933 ISSN: 1465-6566
- WEINSTOCK-GUTTMAN B ET AL: "COMBINATION THERAPY FOR MULTIPLE SCLEROSIS THE TREATMENT STRATEGY OF THE FUTURE?" CNS DRUGS, ADIS INTERNATIONAL, AUCKLAND, NZ LNKD- DOI:10.2165/00023210-200418120-00003, vol. 18, no. 12, 1 January 2004 (2004-01-01), pages 777-792, XP009056907 ISSN: 1172-7047
- YOUDIM MOUSSA BH: "Rasagiline: an anti-Parkinson drug with neuroprotective activity." EXPERT REVIEW OF NEUROTHERAPEUTICS NOV 2003 LNKD- PUBMED:19810877, vol. 3, no. 6, November 2003 (2003-11), pages 737-749, XP009131381 ISSN: 1744-8360
- BROD S A ET AL: "Combination therapy with glatiramer acetate (copolymer-1) and a type I interferon (IFN-alpha) does not improve experimental autoimmune encephalomyelitis.", ANNALS OF NEUROLOGY JAN 2000 LNKD- PUBMED:10632113, vol. 47, no. 1, January 2000 (2000-01), pages 127-131, ISSN: 0364-5134
- YONG V WEE: "Differential mechanisms of action of interferon-beta and glatiramer aetate in MS.", NEUROLOGY 24 SEP 2002 LNKD- PUBMED:12349849, vol. 59, no. 6, 24 September 2002 (2002-09-24), pages 802-808, ISSN: 0028-3878

## Description

### Field of the Invention

The subject invention relates to combination therapy for treating multiple sclerosis.

### Background of the Invention

One of the more common neurologic diseases in human adults is multiple sclerosis. This condition is a chronic, inflammatory CNS disease characterized pathologically by demyelination. There are five main forms of multiple sclerosis: 1) benign multiple sclerosis; 2) relapsing-remitting multiple sclerosis (RR-MS); 3) secondary progressive multiple sclerosis (SP-MS); 4) primary progressive multiple sclerosis (PP-MS); and 5) progressive-relapsing multiple sclerosis (PR-MS). Benign multiple sclerosis is characterized by 1-2 exacerbations with complete recovery, no lasting disability and no disease progression for 10-15 years after the initial onset. Benign multiple sclerosis may, however, progress into other forms of multiple sclerosis. Patients suffering from RR-MS experience sporadic exacerbations or relapses, as well as periods of remission. Lesions and evidence of axonal loss may or may not be visible on MRI for patients with RR-MS. SP-MS may evolve from RR-MS. Patients afflicted with SP-MS have relapses, a diminishing degree of recovery during remissions, less frequent remissions and more pronounced neurological deficits than RR-MS patients. Enlarged ventricles, which are markers for atrophy of the corpus callosum, midline center and spinal cord, are visible on MRI of patients with SP-MS. PP-MS is characterized by a steady progression of increasing neurological deficits without distinct attacks or remissions. Cerebral lesions, diffuse spinal cord damage and evidence of axonal loss are evident on the MRI of patients with PP-MS. PR-MS has periods of acute exacerbations while proceeding along a course of increasing neurological deficits without remissions. Lesions are evident on MRI of patients suffering from PR-MS (Multiple sclerosis: its diagnosis, symptoms, types and stages, 2003 <http://www.albany.net/∼tjc/multiple-sclerosis.html>).

Researchers have hypothesized that multiple sclerosis is an autoimmune disease (Compston, Genetic susceptibility to multiple sclerosis, in McAlpine's Mutiple Sclerosis, Matthews, B. ed., London: Churchill Livingstone, 1991, 301-319; Hafler and Weiner, MS: A CNS and systemic autoimmune disease, Immunol. Today, 1989, 10:109-107; Olsson, Immunology of multiple sclerosis, Curr. Opin. Neurol. Neurosurg., 1992, 5:195-202). An autoimmune hypothesis is supported by the experimental allergic encephalomyelitis (EAE) model of multiple sclerosis, where the injection of certain myelin components into genetically susceptible animals leads to T cell-mediated CNS demyelination (Parkman, Graft-versus-host Disease, Ann. Rev. Med., 1991, 42: 189-197). Another theory regarding the pathogenesis of multiple sclerosis is that a virus, bacteria or other agent, precipitates an inflammatory response in the CNS, which leads to either: direct or indirect ("bystander") myelin destruction, potentially with an induced autoimmune component (Lampert, Autoimmune and virus-induced demyelinating diseases. A review, Am. J. Path., 1978, 91:176-208; Martyn, The epidemiology of multiple sclerosis, in McAlpine's Multiple Sclerosis, Matthews, B., ed., London: Churchil Livingstone, 1991, 3-40). Another experimental model of multiple sclerosis, Theiler's murine encephalomyelitis virus (TMEV) (Dal Canto, M.C., and H.L. Lipton. 1977. Multiple sclerosis. Animal model: Theiler's virus infection in mice. Am. J. Path. 88:497-500; Rodriguez, M. et al. 1987. Theiler's murine encephalomyelitis: a model of demyelination and persistence of virus. Crit. Rev. Immunol., 7:325), supports the theory that a foreign agent initiates multiple sclerosis. In the TMEV model, injection of the virus results in spinal cord demyelination.

Glatiramer acetate (GA), also known as Copolymer-1, has been shown to be effective in treating multiple sclerosis (MS) (Lampert, Autoimmune and virus-induced demyelinating diseases. A review, Am. J. Path., 1978, 91:176-208). Daily subcutaneous injections of glatiramer acetate (20 mg/injection) reduce relapse rates, progression of disability, appearance of new lesions by magnetic resonance imaging (MRI), (Johnson et al., Copolymer 1 reduces relapse rate and improves disability in relapsing-remitting multiple sclerosis: results of a phase III multicenter, double-blind placebo-controlled trial. The Copolymer 1 Multiple Sclerosis Study Group, Neurol., 1995, 45:1268.) and appearance of "black holes" (Filippi et al., Glatiramer acetate reduces the proportion of MS lesions evolving into black holes, Neurol., 2001, 57:731-733).

COPAXONE® is the brand name for a formulation containing glatiramer acetate as the active ingredient. Glatiramer acetate is approved for reducing the frequency of relapses in relapsing-remitting multiple sclerosis. Glatiramer acetate consists of the acetate salts of synthetic polypeptides containing four naturally occurring amino acids: L-glutamic acid, L-alanine, L-tyrosine, and L-lysine with an average molar fraction in COPAXONE® of 0.141, 0.427, 0.095 and 0.338, respectively. In COPAXONE®, the average molecular weight of the glatiramer acetate is 4,700-11,000 daltons. Chemically, glatiramer acetate is designated L-glutamic acid polymer with L-alanine, L-lysine and L-tyrosine, acetate (salt). Its structural formula is:
(Glu, Ala, Lys, Tyr)ₓCH₃COOH
(C₅H₉NO₄·C₃H₇NO₂·C₆H₁₄N₂O₂·C₉H₁₄NO₃)_{χ}·χC₂H₄O₂

### CAS - 147245-92-9.

The recommended dosing schedule of COPAXONE® for relapsing-remitting multiple sclerosis is 20 mg per day injected subcutaneously ("COPAXONE®" in Physician's Desk Reference, Medical Economics Co., Inc., Montvale, NJ, 2003, 3214-3218; see also U.S. Patent No. 3,849,550, issued November 19, 1974 to Teitelbaum, et al.; U.S. Patent No. 5,800,808, issued September 1, 1998 to Konfino, et al.; U.S. Patent No. 5,858,964, issued January 12, 1999 to Aharoni, et al.; U.S. Patent No. 5,981,589, issued November 9, 1999 to Konfino, et al.; U.S. Patent No. 6,048,898, issued April 11, 2000 to Konfino, et al.; U.S. Patent No. 6,054,430, issued April 25, 2000 to Konfino, et al.; U.S. Patent No. 6,214,791, issued April 10, 2001 to Arnon, et al.; U.S. Patent No. 6,342,476, issued January 29, 2002 to Konfino, et al.; U.S. Patent No. 6,362,161, issued March 26, 2002 to Konfino et al., all of which are hereby incorporated by reference).

Rasagiline has the chemical name R(+)-N-propargyl-1-aminoindan and its structural formula is:

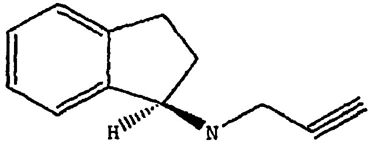

Rasagiline has been shown to be effective in stroke models (Speiser Z. et al.; Eliash S. et al.) and in models of traumatic head injury (Huang W. et al.). Rasagiline, its salts, preparation and use for the treatment of Parkinson's disease, memory disorders and other neurological disorders have been the subject of numerous patents, including U.S. Patent No. 5,387,612, issued February 7, 1995 to Youdim et al., U.S. Patent No. 5,453,446, issued September 26, 1995 to Youdim et al.; U.S. Patent No. 5,457,133, issued October 10, 1995 to Youdim et al.; U.S. Patent No. 5,519,061, issued May 21, 1996 to Youdim et al.; U.S. Patent No. 5,532,415, issued July 2, 1996 to Youdim et al.; U.S. Patent No. 5,576,353, issued November 19, 1996 to Youdim et al.; U.S. Patent No. 5,599,991, issued February 4, 1997 to Youdim et al.; U.S. Patent No. 5,668,181, issued September 16, 1997 to Youdim et al.; U.S. Patent No. 5,786,390, issued July 28, 1998 to Youdim et al.; U.S. Patent No. 5,519,061, issued May 21, 1996 to Youdim et al.; U.S. Patent No. 5,891,923, issued April 6, 1999 to Youdim et al.; U.S. Patent No. 5,744,500, issued April 28, 1998 to Youdim et al. and U.S. Patent No. 6,316,504, issued November 13, 2002 to Youdim et al., the contents of which are incorporated by reference. US 6,316,504 describes the use of rasagiline for treating a list of diseases including multiple sclerosis.

The administration of two drugs to treat a given condition, such as a form of multiple sclerosis, raises a number of potential problems. *In vivo* interactions between two drugs are complex. The effects of any single drug are related to its absorption, distribution, and elimination. When two drugs are introduced into the body, each drug can affect the absorption, distribution, and elimination of the other and hence, alter the effects of the other. For instance, one drug may inhibit, activate or induce the production of enzymes involved in a metabolic route of elimination of the other drug (Guidance for Industry. In vivo drug metabolism/drug interaction studies - study design, data analysis, and recommendations for dosing and labeling, U.S. Dept. Health and Human Svcs., FDA, Ctr. for Drug Eval. and Res., Ctr. for Biologics Eval. and Res., Clin./Pharm., Nov. 1999 <http://www.fda.gov/cber/gdlns/metabol.pdf>). Thus, when two drugs are administered to treat the same condition, it is unpredictable whether each will complement, have no effect on, or interfere with, the therapeutic activity of the other in a human subject.

Not only may the interaction between two drugs affect the intended therapeutic activity of each drug, but the interaction may increase the levels of toxic metabolites (Guidance for Industry. In vivo drug metabolism/drug interaction studies - study design, data analysis, and recommendations for dosing and labeling, U.S. Dept. Health and Human Svcs., FDA, Ctr. for Drug Eval. and Res., Ctr. for Biologics Eval. and Res., Clin./Pharm., Nov. 1999 <http://www.fda.gov/cber/gdlns/metabol.pdf>) . The interaction may also heighten or lessen the side effects of each drug. Hence, upon administration of two drugs to treat a disease, it is unpredictable what change will occur in the negative side profile of each drug.

Additionally, it is accurately difficult to predict when the effects of the interaction between the two drugs will become manifest. For example, metabolic interactions between drugs may become apparent upon the initial administration of the second drug, after the two have reached a steady-state concentration or upon discontinuation of one of the drugs (Guidance for Industry. In vivo drug metabolism/drug interaction studies - study design, data analysis, and recommendations for dosing and labeling, U.S. Dept. Health and Human Svcs., FDA, Ctr. for Drug Eval. and Res., CLr. for Biologics Eval. and Res., Clin./Pharm., Nov. 1999 <http://www.fda.gov/cber/gdins/metabol.pdf>).

Thus, the success of one drug or each drug alone in an *in vitro* model, an animal model, or in humans, may not correlate into efficacy when both drugs are administered to humans.

Attempts to develop and demonstrate an effective combination therapy for multiple sclerosis have been disadvantaged by a lack of control groups to determine if patients who received combination therapy would have done just as well as those on a monotherapy; currently there is no proven combination therapy protocol for the treatment of multiple sclerosis (Weinstock-Guttman and Bakshi, Combination Therapy for Multiple Sclerosis The Treatment Strategy of the Future?, CNS Drugs, 2004, 18(12):777-792).

In accordance with the subject invention, glatiramer acetate and rasagiline are effective in combination to treat a form of multiple sclerosis, specifically, relapsing-remitting multiple sclerosis.

### Summary of the Invention

The subject invention provides glatiramer acetate in combination with rasagiline for use in a method of treating a subject afflicted with a form of multiple sclerosis comprising periodically administering to the subject an amount of glatiramer acetate and an amount of rasagiline or a pharmaceutically acceptable salt thereof, wherein the amounts when taken together are effective to alleviate a symptom of the form of multiple sclerosis in the subject so as to thereby treat the subject.

In addition, the subject invention provides a package comprising
i) a first pharmaceutical composition comprising an amount of glatiramer acetate and a pharmaceutically acceptable carrier;
ii) a second pharmaceutical composition comprising an amount of rasagiline or a pharmaceutically acceptable salt thereof and a pharmaceutically acceptable carrier; and
iii) instructions for use of the first and second pharmaceutical compositions together to alleviate a symptom of a form of multiple sclerosis in a subject.

Further embodiments of the invention are specified in the claims.

### Detailed Description of the Invention

The subject invention provides glatiramer acetate in combination with rasagiline for use in a method of treating a subject afflicted with a form of multiple sclerosis comprising periodically administering to the subject an amount of glatiramer acetate and an amount of rasagiline or a pharmaceutically acceptable salt thereof, wherein the amounts when taken together are effective to alleviate a symptom of the form of multiple sclerosis in the subject so as to thereby treat the subject.

The pharmaceutically acceptable salt of rasagiline may be any pharmaceutically acceptable salt, such as those disclosed by Remington, The Science and Practice of Pharmacy, 20th ed., A. Gennaro et al., eds., Lippincott Williams and Wilkins, Philadelphia, PA, 2000, 704-712. Pharmaceutically acceptable salts of rasagiline include the maleate, fumarate, tartrate, hydrochloride, hydrobromide, esylate, p-toluenesulfonate, benzoate, acetate, phosphate, sulfate, mesylate, esylate, sulfate, or ethylsulfonate salt of rasagiline. In a preferred embodiment, the pharmaceutically acceptable salt of rasagiline is the mesylate salt.

In one embodiment, the form of multiple sclerosis is relapsing-remitting multiple sclerosis.

In another embodiment, the subject is a human being.

In a further embodiment, each of the amount of glatiramer acetate when taken alone, and the amount of rasagiline or the pharmaceutically acceptable salt thereof when taken alone is effective to alleviate the symptom of the form of multiple sclerosis.

In an embodiment, either the amount of glatiramer acetate when taken alone, the amount of rasagiline or the pharmaceutically acceptable salt thereof when taken alone or each such amount when taken alone is not effective to alleviate the symptom of the form of multiple sclerosis.

In yet another embodiment, the symptom is the frequency of relapses, the frequency of clinical exacerbation, or the accumulation of physical disability.

In one embodiment, the amount of glatiramer acetate may be 10 to 80 mg; or 12 to 70 mg; or 14 to 60 mg; or 16 to 50 mg; or 18 to 40 mg; or 20 to 30 mg; or 20 mg.

Alternatively, the amount of glatiramer acetate may be in the range from 10 to 600 mg/week; or 100 to 550 mg/week; or 150 to 500 mg/week; or 200 to 450 mg/week; or 250 to 400 mg/week; or 300 to 350 mg/week; or 300 mg/week.

In another embodiment, the amount of glatiramer acetate may be in the range from 50 to 150 mg/day; or 60 to 140 mg/day; or 70 to 130 mg/day; or 80 to 120 mg/day; or 90 to 110 mg/day; or 100 mg/day.

Alternatively, the amount of glatiramer acetate may be in the range from 10 to 80 mg/day; or 12 to 70 mg/day; or 14 to 60 mg/day; or 16 to 50 mg/day; or 18 to 40 mg/day; or 19 to 30 mg/day; or 20 mg/day.

For each amount of glatiramer acetate, the amount of rasagiline or the pharmaceutically acceptable salt thereof may be 0.01-100 mg/day; 0.01-80 mg/day; or 0.025-60 mg/day; or 0.05-40 mg/day; or 0.075-20 mg/day; or 0.1-10 mg/day; or 0.25-7.5 mg/day; or 0.5-5.0 mg/day; or 0.75-2.5 mg/day; or 1-2 mg/day; or 2 mg/day.

In another alternative, the amount of rasagiline or the pharmaceutically acceptable salt thereof may be from 0.5 mg/kg body weight of the subject per administration to 2.5 mg/kg body weight of the subject per administration; or 0.75 mg/kg body weight of the subject per administration to 2.25 mg/kg body weight of the subject per administration; or 1.0 mg/kg body weight of the subject per administration to 2.0 mg/kg body weight of the subject per administration; or 1.5 mg/kg body weight of the subject per administration.

In one embodiment, the periodic administration of glatiramer acetate is effected daily.

In another embodiment, the periodic administration of glatiramer acetate is effected twice daily at one half the amount.

In an additional embodiment, the periodic administration of glatiramer acetate is effected once every 3 to 11 days; or once every 5 to 9 days; or once every 7 days; or once every 24 hours.

In a further embodiment, the periodic administration of rasagiline or the pharmaceutically acceptable salt thereof is effected daily.

For each administration schedule of glatiramer acetate, the periodic administration of rasagiline or the pharmaceutically acceptable salt thereof may be effected once every 16-32 hours; or once every 18-30 hours; or once every 20-28 hours; or once every 22-26 hours.

In a further embodiment, the administration of the glatiramer acetate substantially precedes the administration of the rasagiline or the pharmaceutically acceptable salt thereof.

In an added embodiment, the administration of the rasagiline or the pharmaceutically acceptable salt thereof substantially precedes the administration of the glatiramer acetate.

In one embodiment, the glatiramer acetate and the rasagiline or the pharmaceutically acceptable salt thereof may be administered for a period of time of at least 4 days. In a further embodiment, the period of time may be 5 days to 5 years; or 10 days to 3 years; or 2 weeks to 1 year; or 1 month to 6 months; or 3 months to 4 months. In yet another embodiment, the glatiramer acetate and the rasagiline or the pharmaceutically acceptable salt thereof may be administered for the lifetime of the subject.

The administration of glatiramer acetate or rasagiline or the pharmaceutically acceptable salt thereof may each independently be oral, nasal, pulmonary, parenteral, intravenous, intra-articular, transdermal, intradermal, subcutaneous, topical, intramuscular, rectal, intrathecal, intraocular, buccal or by gavage. Rasagiline or the pharmaceutically acceptable salt thereof may be administered intravenously, orally, rectally, transdermally, or parenterally. The preferred route of administration for glatiramer acetate is subcutaneous or oral. One of skill in the art would recognize that doses at the higher end of the range may be required for oral administration.

In one embodiment, the administration of the glatiramer acetate may be subcutaneous, intraperitoneal, intravenous, intramuscular, intraocular or oral and the administration of the rasagiline or the pharmaceutically acceptable salt thereof may be oral. In another embodiment, the administration of the glatiramer acetate may be subcutaneous and the administration of the rasagiline or the pharmaceutically acceptable salt thereof may be oral.

The subject invention also provides a package comprising
i) a first pharmaceutical composition comprising an amount of glatiramer acetate and a pharmaceutically acceptable carrier;
ii) a second pharmaceutical composition comprising an amount of rasagiline or a pharmaceutically acceptable salt thereof and a pharmaceutically acceptable carrier; and
iii) instructions for use of the first and second pharmaceutical compositions together to alleviate a symptom of a form of multiple sclerosis in a subject.

In an embodiment of the package, the amount of glatiramer acetate may be in the range from 10 to 600 mg; or 100 to 550 mg; or 150 to 500 mg; or 200 to 450 mg; or 250 to 400 mg; or 300 to 350 mg; or 300 mg.

In another embodiment of the package, the amount of glatiramer acetate may be in the range from 10 to 80 mg; or 12 to 70 mg; or 14 to 60 mg; or 16 to 50 mg; or 18 to 40 mg; or 19 to 30 mg; or 20 mg.

Alternatively, the amount of glatiramer acetate in the package may be in the range from 50 to 150 mg; or 60 to 140 mg; or 70 to 130 mg; or 80 to 120 mg; or 90 to 110 mg; or 100 mg.

For each amount of glatiramer acetate in the package, the amount of rasagiline or the pharmaceutically acceptable salt thereof in the package may be 0.1-100 mg; or 0.1-80 mg; or 0.1-60 mg; or 0.1-40 mg; or 0.1-20 mg; or 0.1-10 mg; or 0.25-8 mg; or 0.5-6 mg; or 0.75-4 mg; or 1-2 mg; or 2 mg.

The subject invention further provides a pharmaceutical combination comprising separate dosage forms of an amount of glatiramer acetate and an amount of rasagiline or the pharmaceutically acceptable salt thereof, which combination is useful to alleviate a symptom of a form of multiple sclerosis in a subject.

In an embodiment of the pharmaceutical combination, each of the amount of glatiramer acetate when taken alone and the amount of rasagiline or the pharmaceutically acceptable salt thereof when taken alone is effective to alleviate the symptom of multiple sclerosis.

In an additional embodiment of the pharmaceutical combination, either of the amount of glatiramer acetate when taken alone, the amount of rasagiline or the pharmaceutically acceptable salt thereof when taken alone or each such amount when taken alone is not effective to alleviate the symptom of multiple sclerosis.

In a further embodiment, the pharmaceutical combination may be for simultaneous, separate or sequential use to treat the form of multiple sclerosis in the subject.

The subject invention further provides for a pharmaceutical composition comprising an amount of glatiramer acetate and an amount of rasagiline, wherein the amounts when taken together are effective to alleviate a symptom of a form of multiple sclerosis in a subject.

In an embodiment of the pharmaceutical composition, the amount of glatiramer acetate when taken alone and the amount of rasagiline when taken alone is effective to alleviate the symptom of multiple sclerosis.

In another embodiment of the pharmaceutical composition, the amount of glatiramer acetate when taken alone, or the amount of rasagiline when taken alone or each such amount when taken alone is not effective to alleviate the symptom of multiple sclerosis.

The subject invention further provides for a product containing glatiramer acetate and rasagiline as a combined preparation for simultaneous, separate or sequential use in therapy.

In another embodiment, the product contains glatiramer acetate and rasagiline as a combined preparation for simultaneous, separate or sequential use in therapy of multiple sclerosis.

The subject invention further provides for the use of glatiramer acetate and rasagiline for the manufacture of a combined perparation medicament for the treatment of multiple sclerosis, wherein glatiramer acetate and rasagiline are administered simultaneously, separately or sequentially.

In an embodiment, the use is sequential at an interval of up to 24 hours.

In another embodiment, the interval is from 1 to 12 hours.

In a further embodiment, the interval is 2 hours.

In an embodiment, the use is separate.

In an embodiment, the use is simultaneous.

The subject invention further provides for the use of rasagiline for the manufacture of a medicament for the treatment of multiple sclerosis in a patient who is being treated with glatiramer acetate for the treatment of multiple sclerosis.

The subject invention further provides for the use of rasagiline for the manufacture of a medicament for the treatment of multiple sclerosis in a patient population that is being treated with glatiramer acetate for the treatment of multiple sclerosis.

The subject invention further provides for the use of rasagiline for the manufacture of a medicament for enhancing the treatment of multiple sclerosis in a patient who is being treated with glatiramer acetate for the treatment of multiple sclerosis.

Formulations of the invention suitable for oral administration may be in the form of capsules, pills, tablets, powders, granules, or as a solution or a suspension in an aqueous or nonaqueous liquid, or as an oil-in-water or water-in-oil liquid emulsion, or as an elixir or syrup, or as pastilles (using an inert base, such as gelatin and glycerin, or sucrose and acacia) and/or as mouth washes and the like, each containing a predetermined amount of the active compound or compounds.

In solid dosage forms of the invention for oral administration (capsules, tablets, pills, dragees, powders, granules and the like), the active ingredient (s) is mixed with one or more pharmaceutically acceptable carriers, such as sodium citrate or dicalcium phosphate, and/or any of the following: fillers or extenders, such as starches, lactose, sucrose, glucose, mannitol, and/or silicic acid; binders, such as, for example, carboxymethylcellulose, alginates, gelatin, polyvinyl pyrrolidone, sucrose and/or acacia; humectants, such as glycerol; disintegrating agents, such as agar-agar, calcium carbonate, calcium phosphate, potato or tapioca starch, alginic acid, certain silicates, and sodium carbonate; solution retarding agents, such as paraffin; absorption accelerators, such as quaternary ammonium compounds; wetting agents, such as, for example, cetyl alcohol and glycerol monostearate; absorbents, such as kaolin and bentonite clay; lubricants, such a talc, calcium stearate, magnesium stearate, solid polyethylene glycols, sodium lauryl sulfate, and mixtures thereof; and coloring agents. In the case of capsules, tablets and pills, the pharmaceutical compositions may also comprise buffering agents. Solid compositions of a similar type may also be employed as fillers in soft and hard-filled gelatin capsules using such excipients as lactose or milk sugars, as well as high molecular weight polyethylene glycols and the like.

Liquid dosage forms for oral administration of the active ingredients include pharmaceutically acceptable emulsions, microemulsions, solutions, suspensions, syrups and elixirs. In addition to the active ingredient(s), the liquid dosage forms may contain inert dilutents commonly used in the art, such as, for example, water or other solvents, solubilizing agents and emulsifiers, such as ethyl alcohol, isopropyl alcohol, ethyl carbonate, ethyl acetate, benzyl alcohol, benzyl benzoate, propylene glycol, 1,3-butylene glycol, oils (in particular, cottonseed, groundnut, corn, germ, olive, castor and sesame oils), glycerol, tetrahydrofuryl alcohol, polyethylene glycols and fatty acid esters of sorbitan, and mixtures thereof.

Suspensions, in addition to the active compounds, may contain suspending agents such as ethoxylated isostearyl alcohols, polyoxyethylene sorbitol and sorbitan esters, microcrystalline cellulose, aluminum metahydroxide, bentonite, agar-agar and tragacanth, and mixtures thereof.

The pharmaceutical compositions, particularly those comprising glatiramer acetate, may also include human adjuvants or carriers known to those skilled in the art. Such adjuvants include complete Freund's adjuvant and incomplete Freund's adjuvant. The compositions may also comprise wetting agents, emulsifying and suspending agents, sweetening, flavoring, coloring, perfuming and preservative agents.

Glatiramer acetate may be formulated into pharmaceutical compositions with pharmaceutically acceptable carriers, such as water or saline and may be formulated into eye drops. Glatiramer acetate may also be formulated into delivery systems, such as matrix systems.

This invention will be better understood from the Experimental Details which follow. However, one skilled in the art will readily appreciate that the specific methods and results discussed are merely illustrative of the invention as described more fully in the claims which follow thereafter.

### Experimental Details

### EXAMPLE 1: CLINICAL TRIAL OF MULTIPLE SCLEROSIS

The purpose of this trial is to compare the treatment of participants with relapsing-remitting multiple sclerosis (RR-MS) with COPAXONE® in combination with rasagiline mesylate, with treatment with COPAXONE® in combination with placebo. The clinical objective is to evaluate the effect of treatments on MRI variables, clinical evaluations and immunological profile.

The design of this trial is a randomized, double-masked, 2-arm study of COPAXONE® in combination with rasagiline mesylate versus COPAXONE® in combination with placebo for the treatment of relapsing-remitting multiple sclerosis. Twenty patients with RR-MS who meet the inclusion/exclusion criteria are enrolled per arm. Patients are randomized and receive either 20 mg SQ (subcutaneous) of COPAXONE® daily plus an oral dose of placebo daily or 20 mg SQ of COPAXONE® in combination with 2 mg oral rasagiline mesylate daily.

Participant inclusion criteria are as follows: 1) men or women age 18 to 50 years; 2) RR-MS according to the guidelines from the International Panel on the Diagnosis of MS (McDonald et al., Recommended diagnostic criteria for multiple sclerosis: guidelines from the International Panel on the diagnosis of multiple sclerosis. Ann. Neurol., 2001, 50:121-127); 3) two separate documented relapses in the last two years; 4) active MRI with at least one gadolinium(Gd)-enhancing lesion in the MRI scan at screening; 5) EDSS (extended disability status scale) score between 1.0 and 5.0; 6) no relapse during screening period; 6) pre-treatment with COPAXONE® for at least three weeks, but no more than four weeks, prior to baseline visit; and 7) ability to understand and provide informed consent. Participant exclusion criteria include the following: 1) normal brain MRI; 2) prior treatment with COPAXONE® other than the scheduled three to four week pretreatment prior to baseline visit; 3) previous treatment with immunomodulating agents such as interferon beta or IVIg for the last 6 months prior to entry; 4) previous use of immunosuppressive agents (including azathioprine) in the last 12 months prior study entry; 5) steroid treatment one month prior to entry; 6) women not willing to practice reliable methods of contraception; 7) pregnant or nursing women; 8) life threatening or clinically significant diseases; 9) history of alcohol and drug abuse within 6 months prior enrollment; 10) known history of sensitivity to Gd; 11) uncontrolled and uncontrollable head movements (tremor, tics, etc.), muscle spasms, significant urinary urgency and claustrophobia, which will prevent the subject from lying still during the MRI scan; and 12) participation in other investigational therapy in the last 90 days.

MRI scans are performed during the screening visit (for eligibility) and at months 5, 10, 11 and 12. Full physical and neurological examinations are performed at screening, baseline and at months 2, 5, 9 and 12. Safety laboratory is performed at screening baseline and at months 1, 2, 5, 9 and 12. In addition, blood Ca⁺ levels are monitored on the first and second months after baseline visit. The immunological profile is monitored at baseline and at months 1, 2, 4, and 5.

Primary efficacy endpoints include the following: 1) MRI variables as measured on months 10, 11, and 12; 2) total number and volume of T1 GD-enhanced lesions; 3) total number of new T2 lesions; and 4) total volume of T2 lesions. Secondary efficacy endpoints encompass the following: 1) changes in immunological parameters; and 2) PBMC proliferation in response to GA in vitro. The tertiary efficacy endpoints are as follows: 1) change from baseline in relapse rate and MS Functional Composite Score (MSFC); and 2) brain atrophy. Tolerability is evaluated with reference to the following: 1) percentage of subjects who discontinue the study; and 2) percentage of subjects who discontinue the study due to adverse events. Safety is evaluated with reference to 1) adverse event frequency and severity; 2) changes in vital signs and 3) clinical laboratory values.

Patients treated with the COPAXONE® and rasagiline mesylate combination exhibit a comparable or greater reduction in T1 and T2 Gd-enhancing lesions and other lesions, as compared to the group receiving COPAXONE® and placebo. Additionally, the group receiving the COPAXONE® and rasagiline mesylate combination demonstrate a comparable or greater reduction in the number of relapses per year as compared with the group receiving COPAXONE® and placebo.

## Claims

1. A pharmaceutical composition comprising an amount of glatiramer acetate and an amount of rasagiline or a pharmaceutically acceptable salt thereof for alleviating a symptom of a form of multiple sclerosis in a subject by periodically administering the pharmaceutical composition to the subject.

2. A pharmaceutical composition comprising an amount of glatiramer acetate for use in alleviating a symptom of a form of multiple sclerosis in a subject in combination with rasagiline or a pharmaceutically acceptable salt thereof by periodically administering the pharmaceutical composition and the rasagiline or a pharmaceutically acceptable salt thereof to the subject.

3. A pharmaceutical composition comprising an amount of rasagiline or a pharmaceutically acceptable salt thereof for use in alleviating a symptom of a form of multiple sclerosis in a subject in combination with glatiramer acetate by periodically administering the pharmaceutical composition and the glatiramer acetate to the subject.

4. A package with compositions i) and ii) for use in alleviating a symptom of a form of multiple sclerosis comprising
i) a first pharmaceutical composition comprising an amount of glatiramer acetate and a pharmaceutically acceptable carrier;
ii) a second pharmaceutical composition comprising an amount of rasagiline or a pharmaceutically acceptable salt thereof and a pharmaceutically acceptable carrier; and
iii) instructions for use of the first and second pharmaceutical compositions together to alleviate a symptom of a form of multiple sclerosis in a subject.

5. The pharmaceutical composition for use according to any one of the claims 1-3 or the package for use according to claim 4, wherein the form of multiple sclerosis is relapsing-remitting multiple sclerosis.

6. The pharmaceutical composition for use according to any one of claims 1-3 or 5 or the package for use according to any one of claims 4-5, wherein the symptom is the frequency of relapses, the frequency of clinical exacerbation, or the accumulation of physical disability.

7. The pharmaceutical composition for use according to any one of claims 1-3 or 5-6 or the package for use according to any one of claims 4-6, wherein the amount of glatiramer acetate is in the range of from 10 to 600 mg/week.

8. The pharmaceutical composition or package for use according to claim 7, wherein the amount of glatiramer acetate is 300 mg/week.

9. The pharmaceutical composition for use according to any one of claims 1-3 or 5-8 or the package for use according to any one of claims 4-8, wherein the amount of glatiramer acetate is in the range of from 50 to 150 mg/day.

10. The pharmaceutical composition or package for use according to claim 9, wherein the amount of glatiramer acetate is 100 mg/day.

11. The pharmaceutical composition for use according to any one of claims 1-3 or 5-6 or the package for use according to any one of claims 4-6, wherein the amount of glatiramer acetate is in the range of 10 to 80 mg/day.

12. The pharmaceutical composition or package for use according to claim 11, wherein the amount of glatiramer acetate is 20 mg/day.

13. The pharmaceutical composition for use according to any one of claims 1-3 or 5-12 or the package for use according to any one of claims 4-12, wherein the amount of rasagiline or a pharmaceutically acceptable salt thereof is in the range from 0.01 mg/day to 100 mg/day.

14. The pharmaceutical composition or package for use according to claim 13, wherein the amount of rasagiline or a pharmaceutically acceptable salt thereof is 2mg/day.

15. The pharmaceutical composition for use according to any one of claims 2, 3 or 5-14, wherein the periodic administration of glatiramer acetate is effected daily.

16. The pharmaceutical composition for use according to any of claims 2, 3 or 5-14, wherein the periodic administration of glatiramer acetate is effected twice daily at one half the amount.

17. The pharmaceutical composition for use according to any of claims 2, 3 or 5-14, wherein the periodic administration of glatiramer acetate is effected once every 5 to 9 days.

18. The pharmaceutical composition for use according to any of claims 2, 3 or 5-17, wherein the administration of the glatiramer acetate substantially precedes the administration of the rasagiline or the pharmaceutically acceptable salt thereof.

19. The pharmaceutical composition for use according to any one of claims 2, 3 or 5-17, wherein the administration of the rasagiline or the pharmaceutically acceptable salt thereof precedes the administration of the glatiramer acetate.

20. The pharmaceutical composition for use according to any of claims 2, 3 of 5-19, wherein the administration of the glatiramer acetate is effected subcutaneously, intraperitoneally, intravenously, intramuscularly, intraocularly or orally and the administration of rasagiline or the pharmaceutically acceptable salt thereof is effected orally.

21. The pharmaceutical composition for use according to claim 20, wherein the administration of the glatiramer acetate is effected subcutaneously and the administration of rasagiline or the pharmaceutically acceptable salt thereof is effected orally.

22. The package for use according to claim 4, wherein the amount of glatiramer acetate is in the range from 10 to 600 mg.

23. The package for use according to claim 22, wherein the amount of glatiramer acetate is 300 mg.

24. The package for use according to claim 22, wherein the amount of glatiramer acetate is 20 mg.

25. The package for use according to any one of claims 4 and 22-24, wherein the amount of rasagiline or the pharmaceutically accepted salt thereof is in the range from 0.1 to 100 mg.

26. The package for use according to claim 25, wherein the amount of rasagiline or the pharmaceutically accepted salt thereof is 2 mg.

27. A product containing glatiramer acetate and rasagiline as a combined preparation for simultaneous, separate or sequential use in therapy.

28. The product for use according to claim 27 for simultaneous, separate or sequential use in therapy of multiple sclerosis.

29. The product for use according to any one of claims 27-28, wherein the use is sequential at an interval of up to 24 hours.

30. The product for use according to claim 29, wherein the interval is from 1 to 12 hours.

31. The product for use according to claim 30, wherein the interval is 2 hours.

32. The product for use according to any one of claims 27-28, wherein the use is separate.

33. The product for use according to any one of claims 27-28, wherein the use is simultaneous.

34. Glatiramer acetate and rasagiline for use in the treatment of multiple sclerosis, wherein the glatiramer acetate and the rasagiline are administered simultaneously, separately or sequentially.

35. The glatiramer acetate and the rasagiline for use according to claim 34, wherein the glatiramer acetate and the rasagiline are administered sequentially at an interval of up to 24 hours.

36. The glatiramer acetate and the rasagiline for use according to claim 35, wherein the interval is from 1 to 12 hours.

37. The glatiramer acetate and the rasagiline for use according to claim 36, wherein the interval is 2 hours.

38. The glatiramer acetate and the rasagiline for use according to claim 34, wherein the glatiramer acetate and the rasagiline are administered separately.

39. The glatiramer acetate and rasagiline for use according to claim 34, wherein the glatiramer acetate and the rasagiline are administered simultaneously.

40. Rasagiline for use in the treatment of multiple sclerosis in a patient who is being treated with glatiramer acetate for the treatment of multiple sclerosis.

41. Rasagiline for use in the treatment of multiple sclerosis in a patient population that is being treated with glatiramer acetate for the treatment of multiple sclerosis.

## Patentansprüche

1. Pharmazeutische Zusammensetzung umfassend eine Menge von Glatirameracetat und eine Menge von Rasagilin oder ein pharmazeutisch verträgliches Salz davon zur Linderung eines Symptoms einer Form von Multiple Sklerose in einem Subjekt durch regelmäßige Verabreichung der pharmazeutischen Zusammensetzung an das Subjekt.

2. Pharmazeutische Zusammensetzung umfassend eine Menge von Glatirameracetat zur Verwendung in der Linderung eines Symptoms einer Form von Multiple Sklerose in einem Subjekt in Kombination mit Rasagilin oder einem pharmazeutisch verträglichen Salz davon durch regelmäßige Verabreichung der pharmazeutischen Zusammensetzung und dem Rasagilin oder einem pharmazeutisch verträglichen Salz davon an das Subjekt.

3. Pharmazeutische Zusammensetzung umfassend eine Menge von Rasagilin oder einem pharmazeutisch verträglichen Salz davon zur Verwendung in der Linderung eines Symptoms einer Form von Multiple Sklerose in einem Subjekt in Kombination mit Glatirameracetat durch regelmäßige Verabreichung der pharmazeutischen Zusammensetzung und dem Glatirameracetat an das Subjekt.

4. Ein Paket mit Zusammensetzungen i) und ii) zur Verwendung in der Linderung eines Symptoms einer Form von Multiple Sklerose umfassend
i) eine erste pharmazeutische Zusammensetzung umfassend eine Menge von Glatirameracetat und einen pharmazeutisch verträglichen Träger;
ii) eine zweite pharmazeutische Zusammensetzung umfassend eine Menge von Rasagilin oder ein pharmazeutisch verträgliches Salz davon und einen pharmazeutisch verträglichen Träger; und
iii) Instruktionen zur Verwendung der ersten und zweiten pharmazeutischen Zusammensetzung, um ein Symptom einer Form von Multiple Sklerose in einem Subjekt zu lindern.

5. Pharmazeutische Zusammensetzung zur Verwendung nach einem der Ansprüche 1 bis 3 oder das Paket zur Verwendung nach Anspruch 4, wobei die Form der Multiplen Sklerose eine schubförmige Multiple Sklerose ist.

6. Pharmazeutische Zusammensetzung zur Verwendung nach einem der Ansprüche 1 bis 3 oder 5 oder das Paket zur Verwendung nach einem der Ansprüche 4 bis 5, wobei das Symptom die Häufigkeit des Rückfalls, die Häufigkeit der klinischen Exazerbation oder die Ansammlung von physischen Behinderungen ist.

7. Pharmazeutische Zusammensetzung zur Verwendung nach einem der Ansprüche 1 bis 3 oder 5 bis 6 oder das Paket zur Verwendung nach einem der Ansprüche 4 bis 6, wobei die Menge an Glatirameracetat in dem Bereich von 10 bis 600 mg/Woche liegt.

8. Pharmazeutische Zusammensetzung oder Paket zur Verwendung nach Anspruch 7, wobei die Menge an Glatirameracetat 300 mg/Woche ist.

9. Pharmazeutische Zusammensetzung zur Verwendung nach einem der Ansprüche 1 bis 3 oder 5 bis 8 oder das Paket zur Verwendung nach einem der Ansprüche 4 bis 8, wobei die Menge an Glatirameracetat in dem Bereich von 50 bis 150 mg/Tag liegt.

10. Pharmazeutische Zusammensetzung oder Paket zur Verwendung nach Anspruch 9, wobei die Menge an Glatirameracetat 100 mg/Tag ist.

11. Pharmazeutische Zusammensetzung zur Verwendung nach einem der Ansprüche 1 bis 3 oder 5 bis 6 oder das Paket zur Verwendung nach einem der Ansprüche 4 bis 6, wobei die Menge an Glatirameracetat in dem Bereich von 10 bis 80 mg/Tag liegt.

12. Pharmazeutische Zusammensetzung oder Paket zur Verwendung nach Anspruch 11, wobei die Menge an Glatirameracetat 20 mg/Tag ist.

13. Pharmazeutische Zusammensetzung zur Verwendung nach einem der Ansprüche 1 bis 3 oder 5 bis 12 oder das Paket zur Verwendung nach einem der Ansprüche 4 bis 12, wobei die Menge an Rasagilin oder einem pharmazeutisch verträglichen Salz davon in dem Bereich von 0,01 mg/Tag bis 100 mg/Tag liegt.

14. Pharmazeutische Zusammensetzung oder Paket zur Verwendung nach Anspruch 13, wobei die Menge an Rasagilin oder einem pharmazeutisch verträglichen Salz davon 2 mg/Tag ist.

15. Pharmazeutische Zusammensetzung zur Verwendung nach einem der Ansprüche 2, 3 oder 5 bis 14, wobei die regelmäßige Verabreichung von Glatirameracetat täglich durchgeführt wird.

16. Pharmazeutische Zusammensetzung zur Verwendung nach einem der Ansprüche 2, 3 oder 5 bis 14, wobei die regelmäßige Verabreichung der halben Menge von Glatirameracetat zweimal täglich durchgeführt wird.

17. Pharmazeutische Zusammensetzung zur Verwendung nach einem der Ansprüche 2, 3 oder 5 bis 14, wobei die regelmäßige Verabreichung von Glatirameracetat einmal jede 5 bis 9 Tage durchgeführt wird.

18. Pharmazeutische Zusammensetzung zur Verwendung nach einem der Ansprüche 2, 3 oder 5 bis 17, wobei die Verabreichung von dem Glatirameracetat im Wesentlichen der Verabreichung von Rasagilin oder dem pharmazeutisch verträglichen Salz davon vorangeht.

19. Pharmazeutische Zusammensetzung zur Verwendung nach einem der Ansprüche 2, 3 oder 5 bis 17, wobei die Verabreichung von dem Rasagilin oder dem pharmazeutisch verträglichen Salz davon der Verabreichung von dem Glatirameracetat vorangeht.

20. Pharmazeutische Zusammensetzung zur Verwendung nach einem der Ansprüche 2, 3 oder 5 bis 19, wobei die Verabreichung von dem Glatirameracetat subkutan, intraperitoneal, intravenös, intramuskulär, intraokular oder oral durchgeführt wird und die Verabreichung von Rasagilin oder dem pharmazeutisch verträglichen Salz davon oral durchgeführt wird.

21. Pharmazeutische Zusammensetzung zur Verwendung nach Anspruch 20, wobei die Verabreichung von dem Glatirameracetat subkutan durchgeführt wird und die Verabreichung von Rasagilin oder dem pharmazeutisch verträglichen Salz davon oral durchgeführt wird.

22. Paket zur Verwendung nach Anspruch 4, wobei die Menge an Glatirameracetat in dem Bereich von 10 bis 600 mg liegt.

23. Paket zur Verwendung nach Anspruch 22, wobei die Menge an Glatirameracetat 300 mg ist.

24. Paket zur Verwendung nach Anspruch 22, wobei die Menge an Glatirameracetat 20 mg ist.

25. Paket zur Verwendung nach einem der Ansprüche 4 und 22 bis 24, wobei die Menge an Rasagilin oder dem pharmazeutisch verträglichen Salz davon in dem Bereich von 0,1 bis 100 mg liegt.

26. Paket zur Verwendung nach Anspruch 25, wobei die Menge an Rasagilin oder dem pharmazeutisch verträglichen Salz davon 2 mg ist.

27. Produkt enthaltend Glatirameracetat und Rasagilin als ein kombiniertes Präparat zur gleichzeitigen, separaten oder sequenziellen Verwendung in der Therapie.

28. Produkt zur Verwendung nach Anspruch 27 zur gleichzeitigen, separaten oder sequenziellen Verwendung in der Therapie von Multiple Sklerose.

29. Produkt zur Verwendung nach einem der Ansprüche 27 bis 28, wobei die Verwendung sequenziell in einem Intervall von bis zu 24 Stunden ist.

30. Produkt zur Verwendung nach Anspruch 29, wobei das Intervall von 1 bis 12 Stunden ist.

31. Produkt zur Verwendung nach Anspruch 30, wobei das Intervall 2 Stunden ist.

32. Produkt zur Verwendung nach einem der Ansprüche 27 bis 28, wobei die Verwendung separat ist.

33. Produkt zur Verwendung nach einem der Ansprüche 27 bis 28, wobei die Verwendung simultan ist.

34. Glatirameracetat und Rasagilin zur Verwendung in der Behandlung von Multiple Sklerose, wobei das Glatirameracetat und das Rasagilin gleichzeitig, separat oder sequenziell verabreicht werden.

35. Glatirameracetat und Rasagilin zur Verwendung nach Anspruch 34, wobei das Glatirameracetat und das Rasagilin sequenziell in einem Intervall von bis zu 24 Stunden verabreicht werden.

36. Glatirameracetat und Rasagilin zur Verwendung nach Anspruch 35, wobei das Intervall von 1 bis 12 Stunden ist.

37. Glatirameracetat und Rasagilin zur Verwendung nach Anspruch 36, wobei das Intervall 2 Stunden ist.

38. Glatirameracetat und Rasagilin zur Verwendung nach Anspruch 34, wobei das Glatirameracetat und das Rasagilin separat verabreicht werden.

39. Glatirameracetat und Rasagilin zur Verwendung nach Anspruch 34, wobei das Glatirameracetat und das Rasagilin gleichzeitig verabreicht werden.

40. Rasagilin zur Verwendung in der Behandlung von Multiple Sklerose in einem Patienten, der mit Glatirameracetat behandelt wird, zur Behandlung von Multiple Sklerose.

41. Rasagilin zur Verwendung in der Behandlung von Multiple Sklerose in einer Patientenpopulation, die mit Glatirameracetat behandelt wird, zur Behandlung von Multiple Sklerose.

## Revendications

1. Composition pharmaceutique comprenant une quantité d'acétate de glatiramère et une quantité de rasagiline ou un sel pharmaceutiquement acceptable de celui-ci pour atténuer un symptôme d'une forme de sclérose en plaques chez un sujet par administration périodique de la composition pharmaceutique au sujet.

2. Composition pharmaceutique comprenant une quantité d'acétate de glatiramère à utiliser pour soulager un symptôme d'une forme de sclérose en plaques chez un sujet en combinaison avec la rasagiline ou un sel pharmaceutiquement acceptable de celui-ci par l'administration périodique de la composition pharmaceutique et la rasagiline ou un sel pharmaceutiquement acceptable de celui-ci au sujet.

3. Composition pharmaceutique comprenant une quantité de rasagiline ou un sel pharmaceutiquement acceptable de celui-ci pour une utilisation dans le soulagement d'un symptôme d'une forme de sclérose en plaques chez un sujet en combinaison avec de l'acétate de glatiramère par l'administration périodique de la composition pharmaceutique et l'acétate de glatiramère au sujet.

4. Emsemble avec des compositions i) et ii) pour une utilisation pour soulager un symptôme d'une forme de sclérose en plaques, comprenant
i) une première composition pharmaceutique comprenant une quantité d'acétate de glatiramère et un support pharmaceutiquement acceptable;
ii) une seconde composition pharmaceutique comprenant une quantité de rasagiline ou un de ses sels pharmaceutiquement acceptables et un support pharmaceutiquement acceptable, et
iii) des instructions pour l'utilisation conjointe des première et seconde compositions pharmaceutiques en vue d'atténuer un symptôme d'une forme de sclérose en plaques chez un sujet.

5. Composition pharmaceutique pour une utilisation selon l'une quelconque des revendications 1-3 ou ensemble pour une utilisation selon la revendication 4, où la forme de sclérose en plaques est la forme cyclique de sclérose en plaques.

6. Composition pharmaceutique pour une utilisation selon l'une quelconque des revendications 1-3 ou 5 ou ensemble pour une utilisation selon l'une quelconque des revendications 4-5, où le symptôme est la fréquence des poussées, la fréquence de l'exacerbation clinique, ou l'accumulation de handicap physique.

7. Composition pharmaceutique pour une utilisation selon l'une quelconque des revendications 1-3 ou 5-6 ou ensemble pour une utilisation selon l'une quelconque des revendications 4-6, où la quantité d'acétate de glatiramère est dans la plage de 10 à 600 mg / semaine.

8. Composition pharmaceutique ou ensemble pour une utilisation selon la revendication 7, où la quantité d'acétate de glatiramère est de 300 mg / semaine.

9. Composition pharmaceutique pour une utilisation selon l'une quelconque des revendications 1-3 ou 5-8 ou ensemble pour une utilisation selon l'une quelconque des revendications 4-8, où la quantité d'acétate de glatiramère est dans la plage de 50 à 150 mg / jour.

10. Composition pharmaceutique ou ensemble pour une utilisation selon la revendication 9, où la quantité d'acétate de glatiramère est de 100 mg / jour.

11. Composition pharmaceutique pour une utilisation selon l'une quelconque des revendications 1-3 ou 5-6 ou ensemble pour une utilisation selon l'une quelconque des revendications 4-6, où la quantité d'acétate de glatiramère est dans la plage de 10 à 80 mg / jour.

12. Composition pharmaceutique ou ensemble pour une utilisation selon la revendication 11, où la quantité d'acétate de glatiramère est de 20 mg / jour.

13. Composition pharmaceutique pour une utilisation selon l'une quelconque des revendications 1-3 ou 5-12 ou ensemble pour une utilisation selon l'une quelconque des revendications 4-12, où la quantité de rasagiline ou un sel pharmaceutiquement acceptable de celui-ci est dans la plage allant de 0,01 mg / jour à 100 mg / jour.

14. Composition pharmaceutique ou ensemble pour une utilisation selon la revendication 13, où la quantité de rasagiline ou un sel pharmaceutiquement acceptable de celui-ci est 2 mg / jour.

15. Composition pharmaceutique pour une utilisation selon l'une quelconque des revendications 2, 3 ou 5-14, où l'administration périodique de l'acétate de glatiramère est effectuée quotidiennement.

16. Composition pharmaceutique pour une utilisation selon l'une quelconque des revendications 2, 3 ou 5-14, où l'administration périodique de l'acétate de glatiramère est effectuée deux fois par jour à une moitié de la quantité.

17. Composition pharmaceutique pour une utilisation selon l'une quelconque des revendications 2, 3 ou 5-14, où l'administration périodique de l'acétate de glatiramère est effectuée une fois tous les 5 à 9 jours.

18. Composition pharmaceutique pour une utilisation selon l'une quelconque des revendications 2, 3 ou 5-17, où l'administration de l'acétate de glatiramère précède sensiblement à l'administration de la rasagiline ou du sel pharmaceutiquement acceptable de celui-ci.

19. Composition pharmaceutique pour une utilisation selon l'une quelconque des revendications 2, 3 ou 5-17, où l'administration de la rasagiline ou du sel pharmaceutiquement acceptable de celui-ci précède l'administration de l'acétate de glatiramère.

20. Composition pharmaceutique pour une utilisation selon l'une quelconque des revendications 2, 3 de 5-19, dans laquelle l'administration de l'acétate de glatiramère est effectuée par voie sous-cutanée, intrapéritonéale, intraveineuse.
intramusculaire, intra-oculaire ou par voie orale et l'administration de rasagiline ou du sel pharmaceutiquement acceptable de celui-ci est effectuée par voie orale.

21. Composition pharmaceutique pour une utilisation selon la revendication 20, où l'administration de l'acétate de glatiramère est effectuée par voie sous cutanée et l'administration de rasagiline ou le sel pharmaceutiquement acceptable de celui-ci est effectuée par voie orale.

22. Ensemble pour une utilisation selon la revendication 4, où la quantité d'acétate de glatiramère est dans la plage de 10 à 600 mg.

23. Ensemble pour une utilisation selon la revendication 22, où la quantité d'acétate de glatiramère est de 300 mg.

24. Ensemble pour une utilisation selon la revendication 22, où la quantité d'acétate de glatiramère est de 20 mg.

25. Ensemble pour une utilisation selon l'une quelconque des revendications 4 et 22-24, où la quantité de rasagiline ou de sel pharmaceutiquement acceptable de celui-ci est dans la plage de 0,1 à 100 mg.

26. Ensemble pour une utilisation selon la revendication 25, où la quantité de rasagiline ou de sel pharmaceutiquement acceptable de celui-ci est de 2 mg.

27. Produit contenant de l'acétate de glatiramère et de la rasagiline en tant que préparation combinée pour une utilisation simultanée, séparée ou séquentielle en thérapie.

28. Produit pour une utilisation selon la revendication 27 pour une utilisation simultanée, séparée ou séquentielle dans la thérapie de la sclérose en plaques.

29. Produit pour une utilisation selon l'une quelconque des revendications 27-28, où l'utilisation est séquentielle à un intervalle de 24 heures au plus.

30. Produit pour une utilisation selon la revendication 29, où l'intervalle est de 1 à 12 heures.

31. Produit pour une utilisation selon la revendication 30, où l'intervalle est de 2 heures.

32. Produit pour une utilisation selon l'une quelconque des revendications 27-28, où l'utilisation est séparée.

33. Produit pour une utilisation selon l'une quelconque des revendications 27-28, où l'utilisation est simultanée.

34. Acétate de glatiramère et rasagiline pour une utilisation dans le traitement de la sclérose en plaques, où l'acétate de glatiramère et la rasagiline sont administrés simultanément, séparément ou séquentiellement.

35. Acétate de glatiramère et rasagiline pour une utilisation selon la revendication 34, où l'acétate de glatiramère et la rasagiline sont administrés de manière séquentielle à un intervalle de 24 heures au plus.

36. Acétate de glatiramère et rasagiline pour une utilisation selon la revendication 35, où l'intervalle est de 1 à 12 heures.

37. Acétate de glatiramère et la rasagiline pour une utilisation selon la revendication 36, où l'intervalle est de 2 heures.

38. Acétate de glatiramère et la rasagiline pour une utilisation selon la revendication 34, où l'acétate de glatiramère et la rasagiline sont administrés séparément.

39. Acétate de glatiramère et rasagiline pour une utilisation selon la revendication 34, où l'acétate de glatiramère et la rasagiline sont administrés simultanément.

40. Rasagiline pour une utilisation dans le traitement de la sclérose en plaques chez un patient qui est traité avec de l'acétate de glatiramère pour le traitement de la sclérose en plaques.

41. Rasagiline pour une utilisation dans le traitement de la sclérose en plaques chez une population de patients en cours de traitement avec de l'acétate de glatiramère pour le traitement de la sclérose en plaques.
